# FASCICULE DE BREVET EUROPEEN

(11) **EP 0 757 911 B1**
(45) Date de publication et mention de la délivrance du brevet: **09.04.2003**
(21) Numéro de dépôt: 96401614.1
(22) Date de dépôt: 19.07.1996
(51) Int. Cl.: A61K 31/22, A61K 9/48, A61K 47/10

(54) **Nouvelles formulations galéniques du fénofibrate et leurs applications**
Fenofibrathaltige neue galenische Arzneizusammensetzungen und ihre Verwendung
New galenic formulations containing fenofibrate and their applications

(30) Priorité: 27.07.1995 FR 9509142
(43) Date de publication de la demande: 12.02.1997
(73) Titulaire: CLL PHARMA, 06200 Nice (FR)
(72) Inventeur: Laruelle, Claude, 06270 Villeneuve Loubet (FR)
(74) Mandataire: Vialle-Presles, Marie José

(56) Documents cités:
- EP-A- 0 256 933
- EP-A- 0 330 532
- WO-A-82/01649
- FR-A- 2 617 047
- CHEMICAL ABSTRACTS, vol. 112, no. 8, 19 Février 1990 Columbus, Ohio, US; abstract no. 62494, XP002018177 & 5TH CONGR. INT. TECHNOL. PHARM., vol. 3, - 1989 pages 190-199, A. BEN-AMOR ET AL.: "AUGMENTATION OF THE BIOAVAILABILITY OF A HYPOLIPEMIC AGENT FOR INCORPORATION INTO A LIQUID-CONTAINING GEL"

## Description

La présente invention est relative à des formulations de fénofibrate (DCI) soluble présentant une biodisponibilité significativement améliorée après administration orale (« suprabiodisponibilité »), à leur procédé d'obtention ainsi qu'aux médicaments comprenant ces formulations.

Le fénofibrate est une substance utilisée depuis plus de 20 ans dans la plupart des pays du monde pour le traitement des hyperlipidémies, hypercholestérolémies et hypertriglycéridémies endogènes de l'adulte. Le traitement prolongé par le fénofibrate à raison de 300 à 400 mg par jour permet d'obtenir une réduction du cholestérol total de 20 à 25 % et une réduction des taux de triglycérides de 40 à 50 %. Il s'oppose ainsi au développement de l'athérosclérose.

Cette activité clinique est le résultat de l'effet pharmacologique dû à l'abaissement des fractions athérogènes de faible densité (VLDL et LDL cholestérol). Cet effet pharmacologique se traduit par :
- une amélioration de la répartition du cholestérol plasmatique en réduisant le rapport cholestérol total/cholestérol HDL,
- une diminution moyenne de l'uricémie de l'ordre de 25 %,
- un effet antiagrégant plaquettaire.

Le fénofibrate fut tout d'abord mis sur le marché en France sous la marque LIPANTHYL® en 1975, sous la forme de gélules dosées à 100 mg de principe actif, selon la formule :

| | |
|---|---|
| fénofibrate | 100 mg |
| excipients | qsp 250 mg pour une gélule. |

La posologie usuelle était de 3 à 4.gélules par jour, en 3 ou 4 prises, soit 300 à 400 mg de principe actif par jour.

Selon DROUIN et al. [Current Therapeutic Research, 1979, **26**, 3, 357-362], l'effet hypocholestérolémiant du fénofibrate est démontré avec des taux plasmatiques du métabolite circulant -l'acide fénofibrique- allant de moins de 5 µg/ml jusqu'à 35 µg/ml. Il est souhaitable que les taux circulants ne dépassent pas 10 µg/ml.

Dans la mesure où l'on a pu relever des cas d'atteinte hépatique, des troubles digestifs et intestinaux ainsi que des formations de lithiase biliaire, il est hautement souhaitable de réduire à son minimum actif, la dose de fénofibrate ingérée quotidiennement.

Enfin, DROUIN signale que lorsque les patients sont non répondeurs aux effets pharmacologiques du fénofibrate, ceci est dû à la mauvaise absorption du médicament administré.

Un progrès substantiel fut en particulier la mise au point par l'Inventeur, d'une formulation à 250 mg, permettant une seule prise journalière, selon la formule :

| | |
|---|---|
| fénofibrate | 250 mg |
| excipients | qsp 360 mg pour une gélule |
| (saccharose, amidon, polymères métacryliques). | |

Cette formulation a fait l'objet du Brevet français n° 2 494 112, déposé en 1980.

Le Brevet européen n° 256 933, déposé en 1987, au nom d'ETHYPHARM décrit un médicament sous forme de microgranules à base de fénofibrate, chaque granule comportant un noyau neutre, une couche à base de fénofibrate et une couche de protection ; dans la couche à base de fénofibrate, celui-ci est présent sous forme de microparticules cristallines de dimension inférieure ou égale à 50 µm et de préférence de l'ordre de 10 µm.

Une telle forme galénique favorise en particulier l'absorption du fénofibrate dans le tube digestif.

Le Brevet français n° 2 627 696, déposé en 1988, au nom de FOURNIER INNOVATION ET SYNERGIE concerne une nouvelle forme galénique du fénofibrate, qui comporte du fénofibrate et un agent tensioactif solide co-micronisés. Il concerne également le procédé de préparation de cette forme galénique et son utilisation pour améliorer la biodisponibilité *in vivo*.

Cette nouvelle forme galénique est commercialisée sous la marque LIPANTHYL 200®. Elle représente un progrès majeur puisque la posologie journalière qui était de 300 à 400 mg par jour en 3 ou 4 prises est passée successivement à 250 mg par jour en une seule prise, puis à 200 mg par jour en une seule prise.

En conséquence, l'Inventeur s'est donné pour but de pourvoir à une forme galénique à base de fénofibrate sous forme de solution, permettant l'administration orale d'une dose journalière de fénofibrate inférieure à 200 mg, tout en augmentant l'absorption intestinale du fénofibrate (suprabiodisponibilité).

Dans une première étude, BRODIE et al. [Arzneimittel Forschung, 1976, 26, 5, 896-901] ont montré que la dissolution du fénofibrate dans l'huile de tournesol permet d'obtenir une résorption intestinale presque complète alors que lorsque le produit est administré sous forme solide en gélule, le métabolite actif circulant - l'acide fénofibrique- n'est retrouvé dans les urines qu'à raison de 28% lorsque l'administration est faite à jeun : DESAGER et al. [J. Clin. Pharmacol., 1978, **26**, 12, 570-574] ou à raison de 60 % lorsque l'administration est faite au cours d'un repas : WEIL et al. [Drug Metabolism, 1980, **18**, 1, 115-120].

Toutefois, l'utilisation de l'huile de tournesol comme solvant nécessite des volumes trop importants, de l'ordre de 5 ml, qui ne permettent pas la mise en forme dans une capsule de volume convenable et acceptable par les patients.

Le fénofibrate est une molécule lipophile de formule brute C₂₀O₄H₂₁Cl et de poids moléculaire 360 g. La poudre cristalline blanche, de point de fusion 80°C, est pratiquement insoluble dans l'eau (8 mg/litre). Les cristaux de fénofibrate ont une dimension comprise entre 10 et 150 µm.

Les solvants gras usuels tels que les mono-, di- et triglycérides d'acides gras de C₈ à C₁₆ dérivés d'huile végétales n'ont pas permis d'obtenir les solubilités de fénofibrate requises. L'ajout de surfactifs, tels que des glycérides poly-glycosilés, améliore toutefois sensiblement les solubilités.

La présente invention a pour objet une nouvelle formulation de fénofibrate, utile notamment dans le traitement des hypercholestérolémies et hypertriglycéridémies, caractérisée en ce qu'elle contient essentiellement du fénofibrate en solution dans un agent solubilisant, constitué par un surfactif non ionique, l'éther monoéthylique du diéthylène glycol (EMDG).

De façon surprenante, la sélection d'un solvant surfactif tel que l'éther monoéthylique du diéthylène glycol (EMDG) de poids moléculaire 134 g et de formule C₂H₅-O-CH₂-CH₂-O-CH₂-CH₂-OH permet d'obtenir les résultats recherchés, à savoir une dissolution complète du fénofibrate, ce qui a pour conséquence une augmentation significative de la biodisponibilité du fénofibrate, lors de son administration orale, car sa dissolution complète dans l'EMDG permet d'obtenir une résorption intestinale presque complète.

L'EMDG est un produit de synthèse obtenu par condensation de l'oxyde d'éthylène sur l'éthanol et purifié par rectification.

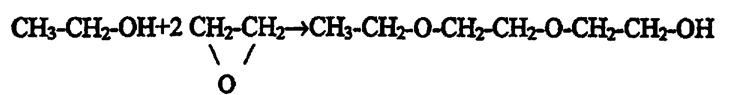

Il est oxydable à l'air et doit être conservé sous atmosphère inerte ou en présence d'antioxydants tels que l'acide ascorbique, le BHT ou le BHA.

L'EMDG est un liquide limpide, de faible viscosité, soluble dans l'eau, amphiphile et partiellement soluble dans les huiles végétales. Il est atoxique et représente un excipient idéal des molécules peu solubles dans l'eau. Son puissant pouvoir de solubilisation des molécules lipophiles est nettement supérieur à celui du propylène glycol, du glycérol ou des polyoxyéthylène glycols classiques tels que PEG 200, 300, 400 etc...

Selon un mode de réalisation avantageux de la solution de fénofibrate, le rapport pondéral agent solubilisant/fénofibrate est compris entre 10 et 20.

Selon un autre mode de réalisation avantageux de la solution de fénofibrate, elle comprend en outre des additifs aptes à augmenter le pouvoir solubilisant de l'EMDG et/ou à augmenter la stabilité de ladite solution.

La présente invention a également pour objet un procédé de solubilisation du fénofibrate, caractérisé en ce qu'il comprend l'incorporation de particules de fénofibrate de dimension inférieure à 10 µm, dans un agent solubilisant, constitué par un surfactif non ionique, l'éther monoéthylique du diéthylène glycol (EMDG). De préférence, le rapport pondéral agent solubilisant/fénofibrate est compris entre 10 et 20.

La présente invention a également pour objet une composition pharmaceutique, destinée à l'administration orale, caractérisée en ce qu'elle contient une quantité efficace d'une solution de fénofibrate telle que définie ci-dessus, incluse dans une capsule molle, de préférence de gélatine.

Une telle composition permet effectivement de réduire la dose de fénofibrate administrée et d'accroître l'absorption intestinale du fénofibrate.

La présente invention a également pour objet un procédé de préparation d'une composition thérapeutique contenant du fénofibrate en solution dans l'éther monoéthylique du diéthylène glycol (EMDG) et présentée en capsule molle, lequel procédé est caractérisé en ce qu'il comprend :
- la micronisation du fénofibrate, de façon à obtenir une poudre dont les particules ont une dimension homogène, inférieure à 10 µm, de préférence de l'ordre de 7 à 10 µm,
- le mélange de ladite poudre avec une solution d'EMDG, jusqu'à complète dissolution du fénofibrate et
- l'incorporation de la solution de fénofibrate dans des capsules molles.

La présente invention a également pour objet l'utilisation de l'éther monoéthylique du diéthylène glycol (EMDG) en tant qu'agent de solubilisation du fénofibrate.

La présente invention a, en outre, pour objet un procédé pour améliorer la biodisponibilité du fénofibrate, caractérisé en ce qu'il comprend la mise en solution du fénofibrate dans l'éther monoéthylique du diéthylène glycol (EMDG).

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'au dessin annexé, dans lequel :

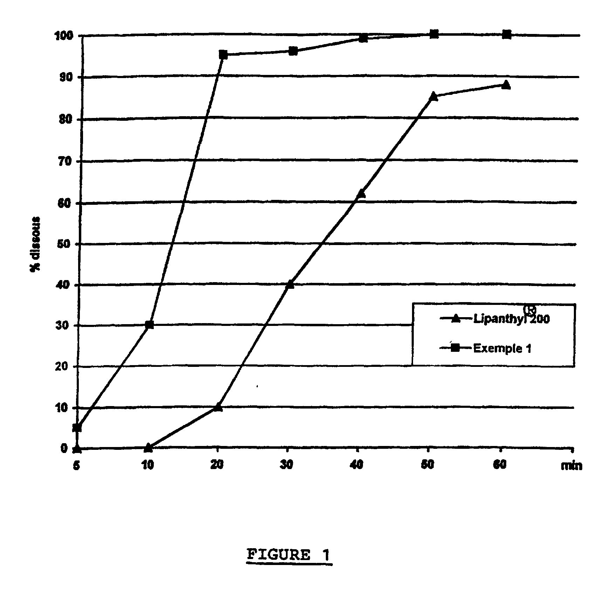

- la figure 1 illustre une étude comparative de la dissolution du fénofibrate avec une composition selon l'invention comparée au LIPANTHYL® 200. Cette figure comporte en abscisse, le temps en minutes et en ordonnée, le pourcentage de fénofibrate dissous.

Il doit être bien entendu, toutefois, que ces exemples sont donnés uniquement à titre d'illustration de l'objet de l'invention, dont ils ne constituent en aucune manière une limitation.

### EXEMPLE 1 : Préparation de capsules molles contenant 50 mg de fénofibrate en solution dans de l'EMDG.

Pour 100 000 capsules molles de gélatine renfermant 50 mg de fénofibrate, la quantité de dissolvant utilisée est la suivante :

| | |
|---|---|
| fénofibrate | 5,0 kg |
| EMDG | 74 kg (ou 75,0 litres de densité 0,989) |

On micronise le fénofibrate dans un appareil à jet d'air de façon à obtenir une poudre dont les particules ont une dimension homogène de l'ordre de 7 à 10 microns afin que la dissolution s'effectue rapidement et dans un délai constant après mélange avec l'EMDG. On obtient une solution limpide et non visqueuse qui constitue la solution médicamenteuse. Les capsules molles sont ensuite fabriquées selon le procédé classique par injection de la solution médicamenteuse et soudure des deux cupules de façon simultanée.

Les capsules molles contiennent 50 mg de fénofibrate dissous dans 0,75 ml d'EMDG. La solution encapsulée dans des capsules transparentes est parfaitement translucide.

Des additifs glycoliques hydromiscibles ainsi que des antioxydants pourront être inclus dans la formule proposée afin d'accroître sensiblement le pouvoir solubilisant de l'EMDG et d'assurer la stabilité de la composition.

La dose unitaire de fénofibrate contenue dans chaque capsule pourra être comprise entre 30 et 200 mg.

### EXEMPLE 2 : Etude de dissolution des capsules molles selon l'exemple 1.

Une étude de dissolution, effectuée selon la méthode de dissolution à

la palette décrite dans la Pharmacopée Européenne 2 éd. « V 5.4 essai de dissolution des formes orales solides », dans les conditions suivantes :
- nature du milieu de dissolution :
   laurylsulfate de sodium 0,1 M
- volume 1 000 ml
- vitesse de rotation 50 t pm
- température du milieu de dissolution 37°C ±0,5°C
- prélèvements 7 ml
- dosage spectrophotométrie UV à 290 nm
a permis de vérifier que les caractéristiques de dissolution étaient les suivantes :

| **Temps de dissolution** **(en min)** | **% dissous Lipanthyl 200®** | **% dissous Exemple 1** |
|---|---|---|
| 5 | 0 | 5 |
| 10 | 0 | 30 |
| 20 | 10 | 95 |
| 30 | 40 | 96 |
| 40 | 62 | 99 |
| 50 | 85 | 100 |
| 60 | 88 | 100 |

Les résultats sont également illustrés à la figure 1.

L'administration de 3 doses successives de 50, 100 et 150 mg de fénofibrate à 6 sujets volontaires sains à jeun a permis de vérifier que la pharmacocinétique de l'acide fénofibrique -métabolite circulant de fénofibrate- était indépendante de la dose dans la gamme de doses étudiées. Cette étude préalable permet de démontrer que l'administration de 100 mg de fénofibrate en capsule molle selon l'invention, en une seule prise, conduit à une concentration plasmatique maximum incluse dans les limites de concentrations considérées comme actives selon DROUIN et al. [Current Therapeutic Research, 1979, **26**, 3, 357-362].

Les taux maximum sont atteints 5 heures après l'administration et les demi-vies d'élimination sont de l'ordre de 20 à 22 heures. Les paramètres pharmaco-cinétiques classiques du fénofibrate sont donc inchangés.

Cependant, la suprabiodisponibilité du fénofibrate, obtenue avec la composition selon l'invention, permet d'obtenir une amélioration significative de la biodisponibilité du fénofibrate, par rapport aux compositions actuellement disponibles.

Le médicament objet de l'invention autorise donc une diminution de la dose journalière administrée. En outre, la présentation soluble montre une absorption intestinale plus homogène entraînant une diminution de la variabilité des taux sanguins inter et intra patients.

En conséquence, la nouvelle formulation galénique de fénofibrate soluble conduit à un médicament remarquable, d'un usage plus aisé et dont les effets secondaires liés à son utilisation sont diminués. La nouvelle formulation permet d'améliorer le confort, l'observance et la compliance du patient.

En outre, contrairement au fénofibrate administré sous forme de poudre, l'administration de capsule molle n'est pas affectée par la présence ou l'absence de nourriture dans l'estomac. L'usage de l'EMDG, solvant hydromiscible, permet un mélange avec les sucs digestifs et conduit à un nappage de la muqueuse intestinale absorbante qui favorise l'absorption du principe actif.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

## Revendications

1. Formulation galénique de fénofibrate, **caractérisée en ce qu'**elle contient essentiellement du fénofibrate en solution dans un agent solubilisant, constitué par un surfactif non ionique, l'éther monoéthylique du diéthylène glycol (EMDG).

2. Formulation galénique de fénofibrate selon la revendication 1, **caractérisée en ce que** le rapport pondéral agent solubilisant/fénofibrate est compris entre 10 et 20.

3. Formulation galénique de fénofibrate selon la revendication 1 ou la revendication 2, **caractérisée en ce qu'**elle comprend en outre des additifs aptes à augmenter le pouvoir solubilisant de l'EMDG et/ou à augmenter la stabilité de ladite solution.

4. Composition pharmaceutique, destinée à l'administration orale, **caractérisée en ce qu'**elle contient une quantité efficace d'une formulation galénique de fénofibrate selon l'une quelconque des revendications 1 à 3, incluse dans une capsule molle, de préférence de gélatine.

5. Procédé de préparation d'une composition thérapeutique selon la revendication 4, **caractérisé en ce qu'**il comprend :
- la micronisation du fénofibrate, de façon à obtenir une poudre dont les particules ont une dimension homogène, inférieure à 10 µm, de préférence de l'ordre de 7 à 10 µm,
- le mélange de ladite poudre avec une solution d'EMDG, jusqu'à complète dissolution du fénofibrate et
- l'incorporation de la solution de fénofibrate dans des capsules molles.

6. Procédé de solubilisation du fénofibrate, **caractérisé en ce qu'**il comprend l'incorporation de particules de fénofibrate de dimension inférieure à 10 µm dans un agent solubilisant, constitué par un surfactif non ionique, l'éther monoéthylique du diéthylène glycol (EMDG).

7. Procédé de solubilisation selon la revendication 6, **caractérisé en ce que** le rapport pondéral agent solubilisant/fénofibrate est compris entre 10 et 20.

8. Utilisation de l'éther monoéthylique du diéthylène glycol (EMDG) en tant qu'agent de solubilisation du fénofibrate.

9. Procédé pour améliorer la biodisponibilité du fénofibrate, **caractérisé en ce qu'**il comprend la mise en solution du fénofibrate dans l'éther monoéthylique du diéthylène glycol (EMDG).

## Patentansprüche

1. Galenische Formulierung von Fenofibrat, **dadurch gekennzeichnet, daß** sie im wesentlichen Fenofibrat in Lösung in einem Lösungsmittel enthält, welches aus einem nichtionischen Tensid, nämlich Diethylenglycolmonoethylether besteht.

2. Galenische Formulierung von Fenofibrat nach Anspruch 1, **dadurch gekennzeichnet, daß** das Gewichtsverhältnis von Lösungsmittel zu Fenofibrat zwischen 10 und 20 beträgt.

3. Galenische Formulierung von Fenofibrat nach Anspruch 1 oder 2, **dadurch gekennzeichnet, daß** sie des weiteren Zusätze enthält, weiche geeignet sind, das Lösungsvermögen von Diethylenglycolmonoethylether und/oder die Stabilität der Lösung zu erhäben.

4. Pharmazeutische Zusammensetzung zur oralen Verabreichung, **dadurch gekennzeichnet, daß** sie eine wirksame Menge einer galenischen Formulierung von Fenofibrat nach einem der Ansprüche 1 bis 3 enthält, welche in eine Weichkapsel, vorzugsweise eine Weichgelatinekapsel, eingehüllt ist.

5. Verfahren zur Herstellung einer therapeutischen Zusammensetzung nach Anspruch 4, **dadurch gekennzeichnet, daß** es umfaßt:
- Mikroniseren von Fenofibrat zur Herstellung eines Pulvers, dessen Partikel eine gleichförmige Größe von weniger als 10 µm, vorzugsweise in der Größenordnung von 7 bis 10 µm, aufweisen,
- Mischen des Pulvers mit einer Diethylenglycolmonoethylether-Lösung bis zur vollständigen Lösung des Fenofibrats, und
- Abfüllen der Fenofibratlösung in Weichkapseln.

6. Verfahren zum Solubilisieren von Fenofibrat, **dadurch gekennzeichnet, daß** es das Einbringen von Fenofibrat-Partikeln mit einer Größe von weniger als 10 µm in ein Lösungsmittel umfaßt, welches aus einem nichtionischen Tensid, nämlich Diethylenglycolmonoethylether besteht.

7. Verfahren zum Solubilisieren nach Anspruch 6, **dadurch gekennzeichnet, daß** das Gewichtsverhältnis von Lösungsmittel zu Fenofibrat zwischen 10 und 20 beträgt.

8. Verwendung von Diethylenglycolmonoethylether als Lösungsmittel für Fenofibrat

9. Verfahren zur Verbesserung der biologischen Verfügbarkeit von Fenofibrat, **dadurch gekennzeichnet, daß** es das Lösen von Fenofibrat in Diethylenglycolmonoethylether umfaßt.

## Claims

1. Fenofibrate pharmaceutical dosage formulation, **characterized in that** it essentially contains fenofibrate in solution in a solubilizing agent consisting of a non-ionic surfactant, diethylene glycol monoethyl ether (DGME).

2. Fenofibrate pharmaceutical dosage formulation according to Claim 1, **characterized in that** the solubilizing agent/fenofibrate ratio by weight is between 10 and 20.

3. Fenofibrate pharmaceutical dosage formulation according to Claim 1 or Claim 2, **characterized in that** it additionally comprises additives capable of increasing the solubilizing power of DGME and/or of increasing the stability of the said solution.

4. Pharmaceutical composition intended for oral adminstration, **characterized in that** it contains an effective amount of a fenofibrate pharmaceutical dosage formulation according to any one of Claims 1 to 3 enclosed in a soft capsule, preferably a soft gelatin capsule.

5. Process for the preparation of a therapeutic composition according to Claim 4, **characterized in that** it comprises:
- the micronization of the fenofibrate, so as to obtain a powder in which the particles have a homogeneous size of less than 10 µm and preferably of the order of 7 to 10 µm,
- mixing the said powder with a DGME solution, until the fenofibrate has completely dissolved, and
- the incorporation of the fenofibrate solution in soft capsules.

6. Process for the solubilization of fenofibrate, **characterized in that** it comprises the incorporation of fenofibrate particles with a size of less than 10 µm in a solubilizing agent consisting of a non-ionic surfactant, diethylene glycol monoethyl ether (DGME).

7. Solubilization process according to Claim 6, **characterized in that** the solubilizing agent/fenofibrate ratio by weight is between 10 and 20.

8. Use of diethylene glycol monoethyl ether (DGME) as solubilization agent for fenofibrate.

9. Process for improving the bioavailability of fenofibrate, **characterized in that** it comprises dissolving fenofibrate in diethylene glycol monoethyl ether (DGME).
